(19) **Europäisches Patentamt European Patent Office Office européen des brevets**

(11) **EP 4 520 190 A1**

(12) **EUROPEAN PATENT APPLICATION**
published in accordance with Art. 153(4) EPC

(43) Date of publication:
**12.03.2025  Bulletin 2025/11**

(21) Application number: **23799373.8**

(22) Date of filing: **05.05.2023**

(51) International Patent Classification (IPC):
**A24B 3/14** *(2006.01)*     **A24F 40/20** *(2020.01)*
**A24F 40/42** *(2020.01)*     **A24B 15/14** *(2006.01)*

(52) Cooperative Patent Classification (CPC):
**A24D 1/20; A24F 40/20; A24F 40/42; A24F 47/00; A61M 15/06**

(86) International application number:
**PCT/IB2023/054720**

(87) International publication number:
**WO 2023/214386 (09.11.2023 Gazette 2023/45)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority:  **06.05.2022  PCT/IB2022/054189**

(71) Applicant: **Compañia Industrial de Tabacos Monte Paz S.A.**
**Montevideo (UY)**

(72) Inventors:
• **GIORDANO PEREIRA, Oscar Alberto**
  **Montevideo (UY)**
• **SPERANZA RODRÍGUEZ, María Soledad**
  **Montevideo (UY)**

(74) Representative: **Herrero & Asociados, S.L.**
**Edificio Aqua - Agustín de Foxá, 4-10**
**28036 Madrid (ES)**

(54) **METHOD FOR PRODUCING REFILL TABLETS FOR HEAT-NOT-BURN (HNB) DEVICES**

(57)     A process for the production of a vapable tablet made of plant-source strands, reconstituted or not, suitable for a fast refill of a Heat-Not-Burn device.

**EP 4 520 190 A1**

## Description

As is well known, official regulations and health concerns have promoted the existence of a series of products that somehow replace cigarettes. The goal is that they do not pose a threat to health and yet still allow enjoying the pleasure of feeling the aroma of tobacco.

These new products have, as expected, their own problems. One of them relates to refilling complications of "Heat-Not-Burn" devices; another is hygiene, and yet another is the difficulty of always achieving an even unit dose when refilling.

Indeed, when refilling a "Heat-Not-Burn" (HnB) type of device it is difficult or nearly impossible to always put the same amount of reconstituted tobacco strands in the receptacle (heater) of the HnB product, to keep at least some of those strands from falling on the floor or other surface, and to clean the receptacle afterwards achieving a similar hygiene.

One factor that aids these three objectives is to present a self-contained refill that is easy to introduce in and remove from the receptacle without affecting the organoleptic properties of the reconstituted tobacco for refill. Obviously, an additional advantage is the ease of transport of these refills and their insertion into the receptacle, as well as the residue disposal.

All of these factors represent a perceived need, and it is with the intention of satisfying such need that this invention is hereby presented.

## Technical field

The present invention relates to the field of the production of aerosols derived from the heating of reconstituted plant-sourced material, either with or without additives. In particular, by way of example and without limitation, the basic approach of the invention is directed to the production of such aerosols for uses of a sensory nature.

## Prior Art

State of the Art (1): Conceptual description

A technical-bibliographic search of applications consistent with the basic approach mentioned above shows that in most cases similar uses refer to aromatherapy, therapeutic or psychoactive purposes and to traditional recipes of non-scientifically proven efficiency. Even in such cases, what is usually used is not the plant itself but its components or products, such as essential oils, oleoresins, alcoholic extracts, etc. One of the systems being notably used currently is that made up of a matrix of tobacco residues or other plant residues from, for example, cocoa shells, residues of plant materials subjected to extraction to the point of exhaustion of components of nutritional interest (e.g. residual grains from beer production), residual husk from coffee curing, etc. The plant matrix that has been reconstituted and eventually treated with additives is hereinafter referred to as **Prepared Plant Material (PPM).**

According to the literature, the usual procedure consists of processing the plant components of the matrix through reconstitution techniques (papermaking methods (US 3,860,012) or castleaf methods (US 5,724,998)) converting the starting materials into a homogeneous sheet with chemical and physical characteristics conditioned by those characteristics of the starting materials and by the incorporation of additives from the reconstitution process that benefit the subsequent behavior. The plant matrix receives an aerosol-generating chemical agent that can be included in the reconstitution process itself or after it. It is also usual to add different types of flavors that give the product its distinctive sensory features. The final material is then handled in its format and presentation according to the intended application.

State of the Art (2): International search

The search included, among others, the terms "plug", "bundle", "reload", "replacement", "heat stick", and "bullet". Both at the beginning and at the end, an in-depth investigation of the commercial supply was performed as an essential way to complement the existing patents or applications.

The search covered "bullets" of both tobacco and any other plant substances.

It should be noted that the overwhelming majority of the supply for HnB devices consists of short, filtered cigarettes called "plugs".

a) Commercial findings

First, we would like to mention the IUOC 2.0 and IUOC 4.0 devices from Yukan Technology Company. They use a normal cigarette with filter, from which the filter is removed. The unfiltered cigarette is introduced at one end of the device, and the filter is introduced at the opposite end of the device. What is striking is that the filter is introduced in a manner, and into a receptacle, similar to the present invention, with the obvious and substantial difference that what is introduced at the mouth end is a filter and not a tobacco "bullet". This completely reverses the invention from a physical and conceptual point of view.

One invention that should be discussed is Ploom TECH, which, although it is for a vapor-operated cigarette, it includes a tobacco bullet. This cigarette produces vapor from an unspecified liquid, passing the vapor through the tobacco and dragging its aroma, literally being a tobacco infusion in a gaseous phase. It is not an HnB system, so it does not fall into the same category, and at the mouth end there is a tobacco bullet of an uncertain composition, which is apparently self-contained in its rigid capsule; it is not tobacco placed in a cavity. See https://www.jti.com/about-us/what-we-do/our-re duced-risk-products#element-4392. At minute 00:18 of the video played at https://www.jti.com/about-us/what-we-do/ our-reduced-risk-products#element--4392, the way in which a capsule is inserted in a manner similar to the Monte Paz bullet, can be observed. Both products are not the same, although their use has certain parallels. The invention is known as PLOOM, by JTI, a member of the Japan Tobacco Group of Companies (JT).

b) Patents

TW201536196, which refers to the insertion in an HnB device of an aroma-generating element, should be mentioned in the first place. It is from Japan Tobacco and there is no translation except for its abstract. They are not devices belonging to the same family, and therefore we estimate that they are not a hindering antecedent.

Other patents that resulted from the search are:

JP1-108967, JP3-90163, JP3-114470. Having examined them, we estimate that they would not constitute an obstacle.

Patents that deserve further analysis are:

- WO2020/089079: this patent, filed by Nerudia Limited relates to: "an HnB consumable comprising an aerosol-forming substrate wherein the aerosol-forming substrate partly comprises a rod of extruded plant material (e.g., extruded tobacco) having an axial bore adapted to receive an external heating element." The basic difference with the present invention consists in the fact that the invention proposed herein lacks the hollow central pipe of that invention, the first and main claims of which are:

  1. An aerosol-forming article comprising an aerosol-forming substrate wherein said substrate at least partly comprises a rod of extruded plant material with a cylindrical cavity in the center having an axial bore adapted to receive an external heating element.
  2. The article according to claim 1 wherein the aerosol-forming substrate comprises a rod of extruded tobacco.

The extruded tobacco may be produced by forming a liquid mixture of powdered tobacco and a binding agent. The liquid mixture is heated and then extruded in a mold to form the cylindrical-shaped extrudate with a hollow central pipe.

According to claim 12 the invention is indeed a refill element for a heat producing device, it being inferred that the invention may not be considered without the filter element or the paper wrapping the substrate and filter.

Therefore, beyond the extruded material or not, the presence of a hollow cylinder in the center entails an important difference with respect to the present invention. So is the statement in the text that the "consumable" may be wrapped with paper and may or may not include a filter. For the reasons above, such substrate is substantially different.

2 = extruded material; 13 = hollow pipe

- WO2019/141373 (PCT/EP2018/051322): No. 4 in the drawing on the front cover is tobacco in a certain and fixed form ("fixed form"). It is estimated that this application will be withdrawn. This is seen only in the internal EPO proceedings, as the patent is in German. It stands out for being a device with a constant diameter along its entire length, which makes it more like a normal cigarette. The tobacco has a fixed shape simply because it is the shape of the cylinder containing it, as clearly stated in point 1.1.1 of the "Written Opinion of the International Search Authority" of the EPO on 21/JUL/2020 ("the tobacco is fixed in its shape by the casing 39"), there being no further proceedings in the dossier after that. This patent refers to the HnB device itself and not to the tobacco refill, which has no shape of its own. Moreover, in the opinion of said Authority, the device is not novel and it is estimated that the patent application will be withdrawn or denied. It has not been published under any other number except PCT/EP2018051322.
- GB2469850: patent filed by British American Tobacco, dated 30/APR/2009. It has "a volatilizing component having a polyester or cellulose acetate matrix containing, in suspension form, a humectant, a polyol, and an active agent such as nicotine. In principle it is a device for using volatilizable substances, but in its second form it accepts tobacco as one of those substances. On page 5, at the end of the first paragraph, it says: "The heat carrying pipe has a prefabricated shaped end 116 to penetrate the tobacco in the volatilizing section 122 to improve contact and heat conduction."

Although this component goes into a particular compartment, its refill is not made with a "bullet" or "wad" as proposed herein, and furthermore the refill has a cylindrical-shaped recess which makes it geometrically different from what is proposed in the present application. The patent refers to an entire device, and not to how to refill it. Therefore, such patent is not considered to be a hindering antecedent, since nowhere does it refer to a small "bale" of reconstituted tobacco compacted or agglomerated in a nonwoven-like style, nor to substances that favor the binding between fibers.

- WO2016/042100: this patent is essentially a method of assembling cigarettes by including three sections in the length of an infinitely repeating cylinder which is then cut into individual cigarettes. It is mentioned herein because in those three joined parts, one part is a substrate capable of forming aerosols. However, there is nothing that suggests that such substrate is similar to the one proposed by Monte Paz, and furthermore, it is always wrapped in a paper that binds the three segments together. Nothing in the claims therein allows thinking of anything similar.

It is not considered to be a hindering antecedent.

- WO2008/015441 (British American Tobacco 02/AUG/2007): HnB device, where the most emphasized feature is the presence of a pipe between the heat source and the volatilizable material, which may contain tobacco. Such material, as stated in Claim 6, has a channel or inlet (recess) which is complementary to the end of the heat pipe, so that the latter enters the former, being number 22 in the attached drawing. Similar to GB2469850, and for the same reasons we estimate that it is not an inhibitory antecedent.

6. A device according to Claim 4 wherein source of volatilizable material includes a channel or recess that is complementary in shape to, and detachably engages, one end of the heat pipe.

There is nothing that allows drawing any conclusions about the conformation of the volatilizable material, "which may contain tobacco", and the replaceable end is simply another "heat stick" with no other particularity than the hollow pipe in about half of its length. It is therefore not a hindering antecedent.

US2014/373856: Invention similar to the invention mentioned hereinabove, wherein its first 12 claims have been cancelled and claim No. 13 became the main claim. It clearly states that the tobacco plug or bullet has cavities or slots for the heating element to be inserted, and in the Figure (No. 5) it informs that the tobacco is wrapped in paper, all of which would mean that it is not agglomerated or extruded, and in addition, it is slotted.

1.-12. (canceled)

13. A smoking article, comprising:

a plurality of elements, including a front-plug and an aerosol-forming substrate,

in which the front-plug defines a hole or slit through which a heating element may be inserted, and

in which the front-plug is substantially cylindrical and has a diameter of 5 mm or greater and a length of at least 2 mm.

/*These paragraphs in the original description written in Spanish include the translation into Spanish of Claim 13 shown hereinabove*/

There are no elements to assume that the tobacco is in an agglomerated form. It certainly is not claimed. In addition, there is the central pipe. Therefore, we estimate that it is not an inhibitory antecedent.

State of the Art (3): Conclusions

We have found no elements that make it possible to assume that the invention proposed herein exists. This includes the world of patents and the commercial field.

General Situation of Insertion of the Invention

We have already seen the state of the art and the commercial situation. We now address the general situation within which our invention will be introduced, as well as its basic characteristics; in other words, its *raison-d'être* as regards to time taken for refill, homogeneity, hygiene, ease of transport, etc.

The use of **PPM** (Prepared Plant Material) for a heating inhalation operation (vaping) requires a device consisting of an adjustable temperature heater, which implies that the user manually handles the product and performs the necessary handling to introduce it properly into the heater with a minimum of waste. These operations generally constitute a waste of time, affect hygiene, they are a source of variability in the sensory appreciation and entail variable time in the consumption of an amount of the product that is necessarily variable given the manual way in which it is taken from its container. Indeed, despite the experience and skill with which the user performs the operation, either by estimating the amount of material introduced into the heater, or by pressing it more or less to adapt it to the heater's geometry, there will always be a certain variation in the inhalation parameters that is transmitted to the variability of flavor, often associated with variations in temperature that can sometimes reach to aggressiveness.

What we propose in the present document is to introduce the PPM into the heater in the form of a porous tablet or capsule with a specific material content of a given weight, calculated based on the desired inhalation profile. The intention is to achieve a consistency in properties similar to that shown by other inhalation systems such as cigarettes, sticks, etc.

The discharge and subsequent cleaning of the heater is also a messy, delicate and time consuming operation. The intention with the present invention is to make that operation simpler, quick and clean.

**Detailed description of the Invention**

In this part of the document we intend to better define the invention from a quantitative, qualitative, and manufacturing point of view.

The present invention consists of a manufacturing process of a fast refill tablet for a "Heat-Not-Burn" device composed of plant source material, processed or not, capable of emitting an inhalable (vapable) aerosol by heating in a suitable device, taking into consideration the requirements mentioned in the introduction of this document for HnB-type products, hereinafter for these purposes referred to as **vapable composite (VC).** Thus, "vapable composite" and "tablet" can be used interchangeably.

The plant source material may be tobacco covering all the usual types of tobacco (virginia or flue-cured, oriental or sun cured, burley or air-cured, dark-cured, american blend, etc.), and their blends and varieties, subjected to reconstitution, roasting, expansion or other processes. The physical form of these tobaccos may be strands with a width between 0.1 mm and 5.0 mm and an average length between 0.2 cm and 4.0 cm, as well as, optionally, polygonal particle shapes sized between 5 and 8 US mesh.

The plant source material should contain one or more aerosol-generating agents, including but not limited to glycerol (G) and propylene glycol (PG) alone or mixed, but maintaining a total percentage in the range of 5% to 40%, more preferably between 10% and 25% (percentages expressed with respect to the plant material). The management of the percentage ratio of G/PG will allow optimizing the aerolization temperature in the range of 120°C to 350°C.

It is therefore pertinent to clarify the formula of the composition of the PPM, i.e. the prepared plant material. This includes as plant material all types of tobaccos, their blends and varieties including reconstituted tobacco, as well as the usual processes that these tobaccos undergo in the industry to achieve their final characteristics. It also includes aerolizing agents such as the mixture of PG and G in the proportion of 5% to 40% or preferably 10% to 25% with respect to the weight of the plant material. If other additives are added (flavors, etc.) which in general are included in small quantities, their weight would be deducted from the weight of plant material and not from the aerosol formers. In summary, the prepared plant material (PPM) consists of:

PPM = Prepared Plant Material (tobaccos, including reconst.) + G/PG + possible additives (flavors, etc.).

Additives may be added to modify flavor or achieve special effects (chemostats, pH modifiers, coolers, etc.).

The final product (VC) may contain binding agents (BA) including but not limited to magnesium stearate, colloidal silicon dioxide (e.g. Aerosil 200), microcrystalline cellulose (e.g. Avicel 200 and/or Adesil PH102), or other similar products known in the pharmaceutical industry. It is worth noting that the binding agents used are of known performance in the pharmaceutical industry, but they have never been used in tablets that are not really tablets, but in a tobacco lattice in strands, permeable, as in this case. This results in a product that provides practicality, ease of use, hygiene, fast and controllable refill, satisfying a need perceived by users of HnB devices.

Some ranges of successful use of binders include proportions of e.g.:

Magnesium stearate: 2 - 50 %.
Colloidal silicon dioxide: 2 - 50 %.
Microcrystalline cellulose: 2 - 60 %
Total binders: 100%

The preparation of the vapable composite itself follows the following equation:

[reconstituted and cut tobacco]    + [chemical treatments]    + [binding agents]
             50-90%                     5-40%

                                                      2-40%


[Prepared Plant Material]    + [binding agents]    =   vapable composite
           60-98%                  2-40%

The vapable composite (VC) is produced by intimately mixing the prepared plant material (PPM) with the binding agents, whereby the ratio to be mixed may include the ranges from 2% BA + 98% PPM to 40% BA + 60% PPM, including all intermediate percentage ratios, but in particular from 20% BA + 80% PPM to 30% BA + 70% PPM. It is understood that the chosen ratio will be one of the determining factors of the amount of plant material (tobacco in our case) that our refill will deliver to the heater of the vaping instrument. This amount of plant material whose vape properties will depend both on the combination of the amounts of Glycerol (G) and Propylene Glycol (PG) and on the ratio between the two, will have a great influence on the time that the refill proposed herein will last to the smoker.

The vapable composite may contain in addition to tobacco, or instead of it, a different plant material such as, but not limited to, cocoa, coffee, eucalyptus, marcela, mint, linden, cannabis, reconstituted tobacco, etc., pre-processed or not. The presence of these plants can be found in any mixture thereof.

The tablet formed by the vapable composite should be easily introduced into a HnB vape device, for which it should have, by way of example but not as limitation, a cylindrical, truncated cone or even spherical shape, with a diameter similar to that of the HnB device and removable by simple gravity from the vape device after having been consumed, having a solid residue of about 80% of the initial weight.

Not to the detriment of the final product itself, it is essential to address the novelty and inventive step of the production process of the Prepared Plant Material (PPM) and how the same is integrated into the final product, vapable composite (VC), which is the result of a very specific method.

Said method to produce a porous tablet of vapable material with a constant constitution, shape, size and weight, suitable to be a fast refill of a Heat-Not-Burn (HnB) device, further ensuring a fast discharge and leaving the vape device clean, said tablet containing a plant part of virginia or flue-cured, oriental or sun cured, burley or air-cured, dark-cured, american blend tobaccos, etc., and their blends and varieties, where the physical form of these tobaccos may be preferably of strands with a width of 0.2 mm to 3.0 mm and an average length of 0.2 cm to 4.0 cm, as well as optionally of particles of polygonal shapes ("scraps" of between 5 and 8 US mesh), includes the following stages:

1. The raw material received, reconstituted tobacco, is subjected to a particle size adjustment process in a deveining machine, forming the PM (plant material), where it undergoes a size degradation to improve the absorption of the treatment and homogenization of the product;

2. The plant material is subjected to a physical reconditioning process in a cylindrical drum having a 45º slope, for a residence time of 2 minutes, which is in turn suitable for the addition of chemical products, and we call it PPM 1, being the application of this treatment at a predetermined flow and temperature, under controlled conditions. During this process the equipment works at 5.5 RPM with a constant flow and steam, and the chemical treatments are applied gradually at a temperature between 30°C and 90°C, in the drum and at a ratio of 30 to 40 (treatment application ratio, given by the final volume of the sauce to be applied per module/batch and its density). Upon exiting the drum, the material has a humidity of between 18% to 35% and after the application of these chemical treatments it is left in a silo to rest during 24 +/- 4 hours, for a better absorption of such chemical treatments, resulting in what we call PPM 1 (Prepared Plant Material 1);

3. PPM 1 is then subjected to a particle size adjustment, working with a cutter at a pressure between 20kN and 60kN and a controlled humidity between 18% and 35%, allowing the production of particles from 0.1 mm to 5.0 mm wide and passing the material at a constant flow; we call it PPM 2,

4. PPM 2 is subjected in the drum dryer to a significant thermal exposure in order to generate the desired reactions. At this point the material is passed through a drum dryer working at a controlled temperature of between 180°C and 260°C during 12 to 20 minutes, at a controlled flow in co-current mode, achieving the characterization of the final product, and allowing the material to exit at a temperature of 35°C to 80°C and with a humidity of between 5% and 18%, depending on its subsequent use; we call it PPM 3;

5. PPM 3 is subjected to a 24-hour stay period;

6. Subsequently, the application of aromatic products will be completed, which due to their sensitivity to heat can only be applied at this point, resulting in PPM 4. At this stage it is done in such a way that it does not modify the physical conditions of the material, taking care that the material has an adequate flow for the correct application of the aromas and that the rotating cylinder does not degrade it,

7. PPM 4 is subjected to a controlled humidity and temperature stay for 15 to 20 days, thus generating PPM 5, where the room is under controlled humidity and temperature conditions so as not to modify the product, with a temperature of 20ºC to 26ºC, and a relative humidity of 60% to 75%;

8. PPM 5 is subjected to a new particle size adjustment and aromatic reinforcement, now called PPM 6, where for this new size adjustment a cutter is used, with a pressure of between 20kN and 60kN, producing particles with a width of 0.2 mm to 4.0 mm and an average length of 0.1 cm to 5.0 cm,

9. PPM 6 is again subjected to a 24-hour stay time in the same or similar chamber as before and with the same controlled temperature and humidity, i.e. a temperature of 20°C to 26°C, and a relative humidity of 60% to 75%;

10. Subsequently, PPM 6 is subjected to a mixing process in a mixing machine operating at 26 RPM and is exposed for 3 minutes to room temperature in order to integrate the group of binding agents and chemicals previously added, thus achieving a homogeneous product between PPM 6 and the binding agents and added products, thus obtaining the Raw Material of the Vapable Composite, referred-to herein as VCRM,

11. Finally, the VCRM is processed in an equipment that provides the final shape of the product, usually using a compression machine like those used in the pharmaceutical industry, at a pressure of 5 to 40kN.

The fast refill is characterized by the fact that the VC (vapable composite) thus obtained has a cylindrical, truncated cone or truncated cone shape with a diameter of between 0.5 and 1.5 cm, a length of 4 to 15 mm and a weight of between 0.2 g and 1.4 g, or any other shape and size that is adaptable to the relevant HnB device.

Another possibility is that the dimensions of these units may be defined according to their final destination, taking into account factors of use such as the size of the heater of the HnB device, the duration time of the tablet, amount of plant material, etc.

It must be pointed out that step 1, carried out in a deveining machine, is not usually used for this type of material, since it is not necessary to devein it as it comes in sheets or plates. The goal is to cause a fragmentation that optimizes the absorption of the treatment and a better size for passing it through the cutting machine (steps 3 and 8).

Step 8, in turn, is in some ways a reprocessing of step 3, unusual in that the already processed material is not usually passed through the cutting machine. Step 8 provides an optimum strand size for the formation of a tablet according to the use for which the present is designed. Each of these steps is inventive, since it is unknown for this particular purpose and used in other processes and for other products, and a person skilled in the art would not ordinarily be inclined to apply them, much less in that order and at that stage of the process. Even less would a person skilled in the art be inclined to combine such steps.

The general dimensions of the refill tablet include a diameter corresponding with the diameter of the HnB device and a length of 4 to 10mm, which may be more.

Thus, in a generic formula for our HnB refill products, the VC vapable compound (composite) will be within the following ranges:

| Product | Limit (%) from | Limit (%) to |
|---|---|---|
| PPM | 60 | 98 |
| [Glycerol and Propylene Glycol (% with respect to plant mass and included in the PPM ] | 5 (pref. 10 | 40 (pref. 25) |
| Binding agents | 2 | 40 |
| Total | 100 | 100 |

In an automatic process, the length and dimensions of the tablet may vary and more than one tablet can be used if the user so prefers, and if the tablets fit in the heater of the device.

Example of a manufactured tablet: it has a cylindrical shape with a diameter of 0.9 +/- 0.2 cm and a height of 0.6 +/- 0.2 cm and a weight of 0.30 +/-0.15 g, which after consumption leaves a residue of 0.24 +/- 0.1 g. It is understood that the described modus operandi will ensure the constancy of the VC that the user will deliver to the vape device for each smoking cycle. Moreover, the refill of the vape device will constitute a smooth operation without loss of vapable material.

Also, the post-vape residue will be discharged compactly from the heater by the simple action of gravity, discharging easily from the heater without the need for special cleaning procedures at the end of each vape/session, much less the use of instruments such as, for example, those used for emptying/cleaning a pipe. This residue is about 80 +/- 15 % of the initial weight of the tablet, depending logically on the physical and chemical constitution of the tablet.

It is worth pointing out the convenience of each individual refill being presented in its own container, which in turn further facilitates refilling.

**Claims**

1. A method for producing a porous tablet of vapable material having a constant constitution, shape, size and weight, suitable as to be a fast refill for a Heat-Not-Burn (HnB) device, further ensuring a fast discharge and leaving the vape device clean, said tablet containing a plant-source tobacco part of virginia or flue-cured, oriental or sun cured, burley or air-cured, dark-cured, american blend, etc. tobaccos, etc., and their blends and varieties, where the physical form of these tobaccos may be preferably of strands having a width of between 0.2 mm and 3.0 mm, and an average length of between 0.2 cm and 4.0 cm, optionally containing as well particles of polygonal shapes ("scraps" with a size between 5 and 8 US mesh), wherein said method includes the following stages:

   - the raw material received, reconstituted tobacco, is subjected to a particle size adjustment process in a deveining machine, forming the PM (plant material), where it undergoes a size degradation improving the absorption of the treatment and homogenization of the product;
   - the PM is subjected to a physical reconditioning process in a cylindrical drum having a 45º slope, for a residence time of 2 minutes, which is in turn suitable for the addition of chemical products, and we call it PPM 1, being the application of this treatment at a predetermined flow and temperature, under controlled conditions. During this process the equipment works at 5.5 RPM with a constant flow and steam, and the chemical treatments are applied gradually at a temperature between 30°C and 90°C, in the drum and at a ratio of 30 to 40 (treatment application ratio, given by the final volume of the sauce to be applied per module/batch and its density). Upon exiting the drum, the material has a humidity of between 18% and 35% and after the application of these chemical treatments it is left in a silos to rest during 24 +/- 4 hours, for a better absorption of such chemical treatments, resulting in what we call PPM 1 (Prepared Plant Material 1);
   - PPM 1 is then subjected to a particle size adjustment, working with a cutter at a pressure between 20kN and 60kN and a controlled humidity between 18% and 35%, allowing the production of particles from 0.1 mm to 5.0 mm wide and passing the material at a constant flow; we call it PPM 2;
   - PPM 2 is subjected in the drum dryer to a significant thermal exposure in order to generate the desired reactions. At this point the material is passed through a drum dryer working at a controlled temperature of between 180°C and 260°C during 12 to 20 minutes, at a controlled flow in co-current mode, achieving the characterization of the final product, and allowing the material to exit at a temperature of 35°C to 80°C and with a humidity of between 5% and 18%, depending on its subsequent use; we call it PPM 3;
   - PPM 3 is subjected to a 24-hour stay period;
   - subsequently, the application of aromatic products will be completed, which due to their sensitivity to heat can only be applied at this point, resulting in PPM 4. At this stage it is done in such a way that it does not modify the physical conditions of the material, taking care that the material has an adequate flow for the correct application of the aromas and that the rotating cylinder does not degrade it,
   - PPM 4 is subjected to a controlled humidity and temperature stay for 15 to 20 days, thus generating PPM 5, where the room is under controlled humidity and temperature conditions so as not to modify the product, with a temperature of 20ºC to 26ºC, and a relative humidity of 60% to 75%;
   - PPM 5 is subjected to a new particle size adjustment and aromatic reinforcement, now called PPM 6, where for this new size adjustment a cutter is used, with a pressure of between 20kN and 60kN, producing particles with a width of 0.2 mm to 4.0 mm and an average length of 0.1 cm to 5.0 cm,
   - PPM 6 is again subjected to a 24-hour stay time in the same or similar chamber as before and with the same controlled temperature and humidity, i.e. a temperature of 20°C to 26°C, and a relative humidity of 60% to 75%;
   - subsequently, PPM 6 is subjected to a mixing process in a mixing machine operating at 26 RPM and is exposed for 3 minutes to room temperature in order to integrate the group of binding agents and chemicals previously added, thus achieving a homogeneous product between PPM 6 and the binding agents and added products, thus obtaining the Raw Material of the Vapable Composite, referred-to herein as VCRM,
   - Finally, the VCRM is processed in an equipment that provides the final shape of the product, usually using a compression machine like those used in the pharmaceutical industry, at a pressure of 5 to 40kN.
   - The fast refill is **characterized by** the fact that the VC (vapable composite) thus obtained has a cylindrical, truncated cone or truncated cone shape with a diameter of between 0.5 and 1.5 cm, a length of 4 to 15 mm and a weight of between 0.2 g and 1.4 g, or any other shape and size that is adaptable to the relevant HnB device. Same claim as 1 wherein the porous tablet contains a plant material other than tobacco such as, by way of example and not limitation, cocoa, coffee, eucalyptus, marcela, mint, linden, cannabis, etc., pre-processed or not.

2. The method of Claim 1, wherein the plant material is other than or in addition to tobacco, such as, for example, either together or separately, cocoa, coffee, eucalyptus, marcela, mint, linden, cannabis, etc., pre-processed or not, the strands of which have the same dimensions as those specified in Claim 1.

3. A tablet which is a fast, stable, constant, uniform and hygienic refill for HnB (Heat-Not-Burn) devices, formed by an agglomeration of strands of reconstituted plant material according to Claim 1, with a width of between 0.2 mm and 4.0 mm, and an average length of between 0.1 cm and 5.0 cm, as well as optionally of polygonal shaped particles ("scraps") of sizes comprised between 5 and 8 US mesh, **characterized in that** those strands or polygonal particles "scraps" of plant material have been produced according to the method of Claims 1 and/or 2.

# INTERNATIONAL SEARCH REPORT

| | |
|---|---|
| International application No. | |
| PCT/IB2023/054720 | |

**A. CLASSIFICATION OF SUBJECT MATTER**

See extra sheet

According to International Patent Classification (IPC) or to both national classification and IPC

**B. FIELDS SEARCHED**

Minimum documentation searched (classification system followed by classification symbols)
A24B, A24F

Documentation searched other than minimum documentation to the extent that such documents are included in the fields searched

Electronic data base consulted during the international search (name of data base and, where practicable, search terms used)

EPODOC, INVENES, WPI, EMBASE, BIOSIS, TXTE, NPL, INTERNET

**C. DOCUMENTS CONSIDERED TO BE RELEVANT**

| Category* | Citation of document, with indication, where appropriate, of the relevant passages | Relevant to claim No. |
|---|---|---|
| Y | CN 113876019 A (CHINA TOBACCO HUBEI IND LLC) 04/01/2022. | 1-3 |
| Y | CN 109846073 A (WUHAN ZUIYIN CHAPIN TEA TECH CO LTD) 07/06/2019.<br><br>CN 112369644 A (HUBEI CHINA TOBACCO IND CO LTD) 19/02/2021. | 1-3 |
| A | CN 101076262 A (BAT CIGARETTENFAB GMBH BAT CIGARETTENFAB GMBH) 21/11/2007. | 1-3 |
| A | | 1-3 |

☒ Further documents are listed in the continuation of Box C.        ☒ See patent family annex.

| | |
|---|---|
| *    Special categories of cited documents:<br>"A"  document defining the general state of the art which is not considered to be of particular relevance.<br>"E"  earlier document but published on or after the international filing date<br>"L"  document which may throw doubts on priority claim(s) or which is cited to establish the publication date of another citation or other special reason (as specified)<br>"O"  document referring to an oral disclosure use, exhibition, or other means.<br>"P"  document published prior to the international filing date but later than the priority date claimed | "T"  later document published after the international filing date or priority date and not in conflict with the application but cited to understand the principle or theory underlying the invention<br>"X"  document of particular relevance; the claimed invention cannot be considered novel or cannot be considered to involve an inventive step when the document is taken alone<br>"Y"  document of particular relevance; the claimed invention cannot be considered to involve an inventive step when the document is combined with one or more other documents , such combination being obvious to a person skilled in the art<br>"&"  document member of the same patent family |
| Date of the actual completion of the international search<br>06/07/2023 | Date of mailing of the international search report<br>**(10/07/2023)** |
| Name and mailing address of the ISA/<br><br>OFICINA ESPAÑOLA DE PATENTES Y MARCAS        Paseo de la Castellana, 75 - 28071 Madrid (España)<br>Facsimile No.: 91 349 53 04 | Authorized officer<br>A. Maquedano Herrero<br><br><br>Telephone No. 913495474 |

Form PCT/ISA/210 (second sheet) (July 2022)

**INTERNATIONAL SEARCH REPORT**

International application No.

PCT/IB2023/054720

| C (continuation). | | DOCUMENTS CONSIDERED TO BE RELEVANT | |
|---|---|---|---|
| Category * | Citation of documents, with indication, where appropriate, of the relevant passages | | Relevant to claim No. |
| A | WO 2022053836 A1 (BRITISH AMERICAN TOBACCO EXPORTS LTD) 17/03/2022, the whole document; in particular: page 1, lines 20-34; page 2, lines 1-6; page 3, line 4 to page 4, line 11; page 5, line 20 a page 6, line 7; page 13, line 20 a page 14, line 3 and claims. | | 1-3 |
| A | US 2019000144 A1 (BLESS ALFRED CHARLES  ET AL.) 03/01/2019, the whole document; in particular, paragraphs [0056] and [0076]. | | 3 |
| P,A | CN 112056610 A (CHINA TOBACCO HENAN IND CO LTD) 11/12/2020. | | 1-3 |

Form PCT/ISA/210 (continuation of second sheet) (July 2022)

# INTERNATIONAL SEARCH REPORT

International application No.

PCT/IB2023/054720

## CLASSIFICATION OF SUBJECT MATTER

*A24B3/14* (2006.01)
*A24F40/20* (2020.01)
*A24F40/42* (2020.01)
*A24B15/14* (2006.01)

Form PCT/ISA/210 (extra sheet) (July 2022)

| INTERNATIONAL SEARCH REPORT | | International application No. | |
|---|---|---|---|
| Information on patent family members | | PCT/IB2023/054720 | |

| Patent document cited in the search report | Publication date | Patent family member(s) | Publication date |
|---|---|---|---|
| CN113876019 A | 04.01.2022 | CN113876019B B | 31.01.2023 |
| CN109846073 A | 07.06.2019 | NONE | |
| CN112369644 A | 19.02.2021 | NONE | |
| CN101076262 A | 21.11.2007 | CL2011001813 A1 | 09.03.2012 |
| | | PL1827142T T3 | 30.11.2011 |
| | | UA89806 C2 | 10.03.2010 |
| | | JP2008522595 A | 03.07.2008 |
| | | JP4912319B B2 | 11.04.2012 |
| | | MY145101 A | 30.12.2011 |
| | | DK1827142T T3 | 05.09.2011 |
| | | ES2364664T T3 | 08.09.2011 |
| | | AT513483T T | 15.07.2011 |
| | | US2011088705 A1 | 21.04.2011 |
| | | US8820328 B2 | 02.09.2014 |
| | | ZA200703169 B | 25.06.2008 |
| | | BRPI0518864 A2 | 16.12.2008 |
| | | BRPI0518864 B1 | 09.04.2013 |
| | | RU2007125629 A | 20.01.2009 |
| | | RU2350233 C1 | 27.03.2009 |
| | | MX2007006929 A | 26.06.2007 |
| | | US2008142027 A1 | 19.06.2008 |
| | | US7934509 B2 | 03.05.2011 |
| | | KR20070087000 A | 27.08.2007 |
| | | KR100967767B B1 | 05.07.2010 |
| | | WO2006061117 A1 | 15.06.2006 |
| | | EP1827142 A1 | 05.09.2007 |
| | | EP1827142 B1 | 22.06.2011 |
| | | DE202004019711UU1 | 14.04.2005 |
| | | DE102004059388 A1 | 14.06.2006 |
| | | DE102004059388 B4 | 30.11.2006 |
| | | CN101076262B B | 23.05.2012 |
| | | CA2584344 A1 | 15.06.2006 |
| | | CA2584344 C | 17.01.2012 |
| | | AU2005313656 A1 | 15.06.2006 |
| | | AU2005313656B B2 | 17.12.2009 |
| | | AR053420 A1 | 09.05.2007 |
| WO2022053836 A1 | 17.03.2022 | KR20230095938 A | 29.06.2023 |
| | | IL301261 A | 01.05.2023 |
| | | IL301260 A | 01.05.2023 |
| | | AU2021339056 A1 | 04.05.2023 |
| | | AU2021341647 A1 | 04.05.2023 |
| | | CZ2021429 A3 | 29.03.2023 |
| | | CZ2021430 A3 | 22.03.2023 |
| | | CA3173898 A1 | 17.03.2022 |
| | | CA3173839 A1 | 17.03.2022 |
| | | RO136078 A2 | 29.11.2022 |
| | | RO136077 A2 | 29.11.2022 |
| | | HU2100322 A1 | 28.05.2022 |

Form PCT/ISA/210 (patent family annex) (July 2022)

## INTERNATIONAL SEARCH REPORT

Information on patent family members

International application No.

PCT/IB2023/054720

| Patent document cited in the search report | Publication date | Patent family member(s) | Publication date |
|---|---|---|---|
| | | HU2100323 A1 | 28.05.2022 |
| | | DK202170451 A1 | 18.03.2022 |
| | | DK202170450 A1 | 18.03.2022 |
| | | SE2151126 A1 | 15.03.2022 |
| | | SE545054 C2 | 14.03.2023 |
| | | SE2151125 A1 | 15.03.2022 |
| | | SE545049 C2 | 07.03.2023 |
| | | PL438909 A1 | 21.03.2022 |
| | | PL438910 A1 | 21.03.2022 |
| | | AT524184 A2 | 15.03.2022 |
| | | AT524183 A2 | 15.03.2022 |
| | | DE102021123239A1 | 17.03.2022 |
| | | DE102021123238A1 | 17.03.2022 |
| | | GB2603828 A | 17.08.2022 |
| | | GB2603569 A | 10.08.2022 |
| | | WO2022053835 A1 | 17.03.2022 |
| US2019000144 A1 | 03.01.2019 | CN115104782 A | 27.09.2022 |
| | | RU2021138426 A | 01.04.2022 |
| | | RU2763406 C1 | 29.12.2021 |
| | | ZA201908613 B | 28.07.2021 |
| | | US2021022402 A1 | 28.01.2021 |
| | | US11684087 B2 | 27.06.2023 |
| | | PH12020500024 A1 | 14.09.2020 |
| | | JP2020525025 A | 27.08.2020 |
| | | BR112019027957A2 | 14.07.2020 |
| | | US2020170304 A1 | 04.06.2020 |
| | | US10834973 B2 | 17.11.2020 |
| | | CN111052857 A | 21.04.2020 |
| | | CN111052857B B | 09.08.2022 |
| | | KR20200026922 A | 11.03.2020 |
| | | CA3068391 A1 | 03.01.2019 |
| | | US10575562 B2 | 03.03.2020 |
| | | EP3646666 A1 | 06.05.2020 |
| | | WO2019003166 A1 | 03.01.2019 |
| CN112056610 A | 11.12.2020 | CN112056610B B | 22.07.2022 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 3860012 A
- US 5724998 A
- TW 201536196
- JP 1108967 A
- JP 3090163 A
- JP 3114470 A
- WO 2020089079 A

- WO 2019141373 A
- EP 2018051322 W
- GB 2469850 A
- WO 2016042100 A
- WO 2008015441 A
- US 2014373856 A